# EUROPÄISCHE PATENTSCHRIFT

(11) **EP 0 374 755 B1**
(45) Veröffentlichungstag und Bekanntmachung des Hinweises auf die Patenterteilung: **19.10.1994**
(21) Anmeldenummer: 89123234.0
(22) Anmeldetag: 15.12.1989
(51) Int. Cl.: A01H 1/02

(54) **Verfahren zur Herstellung von doppelt-haploiden Gurken**
Method to produce double-haploid gerkins
Méthode de production de concombres haploides doubles

(30) Priorität: 22.12.1988 DE 3843199
(43) Veröffentlichungstag der Anmeldung: 27.06.1990
(73) Patentinhaber: NUNHEMS ZADEN BV, NL-6080 AA Haelen (NL)
(72) Erfinder: Dirks, Robert, Dr., B-3680 Maaseik (BE)
(74) Vertreter: Tergau, Ulrich

(56) Entgegenhaltungen:
- EP-A- 0 127 313
- EP-A- 0 171 310
- EP-A- 0 262 971
- Comptes rendus de l'Academie Bulgare des Sciences vol. 38, no. 9, 1985, SofiaSeiten 1243 - 1244; O.A.Dryanovska: "Induced callus in vitro from ovaries andanthers of species of the Cucurbita family"
- H.Kuckuck: "Gartenbauliche Pflanzenzüchtung" 1979, Paul Parey, Berlin;paragrahp 1.3.2.6 Seite 74-78:"Gurken"

## Beschreibung

Die Erfindung betrifft ein mikrobiologisches Verfahren zur Herstellung von doppelt-haploiden Cucumis sativus-Pflanzen durch Kultivierung von haploiden Pflanzen, das wesentlich effektiver als die bisher bekannten Verfahren ist.

Es ist bekannt, daß bei Gurken in niedriger Häufigkeit spontan Haploide gebildet werden, wobei üblicherweise weniger als ein haploider Embryo pro tausend Samen gebildet wird (Aalders 1958; J. Hered. 49,41-44). Diese Häufigkeit kommt bei vielen Pflanzen vor, ist jedoch nicht ausreichend für die Anwendung in Züchtungsverfahren.

Es ist weiterhin bekannt (Truong-André, 1988; Proceedings of the Eucarpia congress on cucurbitaceae, Avignon-Montfavet 31 May - 1-2 June 1988), in vitro haploide Gurken aus extrahierten unbefruchteten Eizellen (Ovulae) 2-6 Wochen nach der Bestäubung der weiblichen Blüten mit bestrahlten Pollen (400-600 gy) zu züchten. Pro 1000 Eier werden etwa 3 haploide lebensfähige Pflanzen erhalten. Auch dieses Verfahren hat also nur sehr begrenzten Wert für die Anwendung in Züchtungsprogrammen.

Die sporadische Bildung von Embryonen und Pflanzen aus Gurken-Staubbeuteln wurde von Sasser und Lazarte; 1982 Hortscience 17,(1) 88 beschrieben, wobei jedoch keine Untersuchungen über die Ploidiestufe gemacht wurden und die Bildung der Pflänzchen höchstwahrscheinlich aus somatischem Gewebe erfolgte.

Callus-Kulturen aus Ovarien und Staubbeuteln wurden von Dryanovska 1985; Comptes Rendus de l'Academie Bulgare des Sciences 38 (9) erhalten, wobei diploide, aneuploide und haploide Metaphasen beobachtet wurden. Die Callus-Bildung soll hierbei nicht nur von Geschlechtszellen ausgehen. Es wurden jedoch keine Pflanzen regeneriert. Diese Beobachtungen liefern somit keinen Beitrag für Züchtungsprogramme.

Es bestand also die Aufgabe, haploide Cucumis sativus-Pflanzen herzustellen und diese - sofern erforderlich - in die doppelt-haploide Pflanze zu überführen. Diese Aufgabe wurde erfindungsgemäß gelöst durch die Isolierung von unreifen oder unbefruchteten Eizellen (Ovulae), Zellen vom Embryosack oder von genannte Zellen enthaltendem ovarialem Gewebe aus Fruchtknoten weiblicher Gurkenblüten, die Induktion der Bildung von Embryonen mit Hilfe eines hormonhaltigen Mediums das bei parthenocarpen Früchten Cytokine und bei nicht parthenocarpen Früchten Auxine enthält, Kultivierung zu haploiden Pflanzen und, falls nicht oder nicht ausreichend spontan erfolgt, Duplizierung des Genoms.

Es handelt sich hierbei also um eine Gynogenese im strengen Sinn ohne jeglichen Einsatz von unfruchtbaren Pollen (induzierte Parthenogenese).

Im folgenden werden bevorzugte Ausführungen des erfindungsgemäßen Verfahrens näher erläutert:
Es ist vorteilhaft, Embryonen aus Ovarien oder Ovarien enthaltendem Fruchtgewebe zu kultivieren. Hierzu werden die Ovarien aus C. sativus-Pflanzen gewonnen, die im Freien oder im Gewächshaus wuchsen, entweder in Erde oder in Hydrokultur. Das Stadium der Ovarien kann von unreifen Ovarien mit geschlossenen Blüten bis zu Ovarien mit reifen offenen Blüten oder sogar mit beginnenden verblühenden Blüten variieren. Bevorzugt ist das Stadium von 1 bis 3 Tagen vor der Öffnung der Blüte.

Die gewonnenen Ovarien werden dekontaminiert, um an ihrer Oberfläche Mikroorganismen abzutöten. Zu dieser Keimfreimachung können übliche und beispielsweise die klassischen Verfahren eingesetzt werden, wie Inkubation in wäßrigen Lösungen von Hypochloritsalzen oder Wasserstoffperoxid für unterschiedliche Zeiten, je nach Größe der Probe. Das Sterilisierungsmittel wird anschließend durch Waschen entfernt oder mit anderen Mitteln inaktiviert. Es ist möglich, aber nicht erforderlich, das umgebende, gegebenenfalls beschädigte Fruchtgewebe zu entfernen; im allgemeinen können die Ovarien ohne weiteres weiterverarbeitet werden. Sie werden hierzu in Stücke geschnitten, die ein oder mehrere unbefruchtete Eizellen enthalten. Der Schnitt kann hierbei in verschiedener Weise erfolgen, beispielsweise longitudinal oder transversal.

Die so erhaltenen Ovarien-Stückchen werden nun zweckmäßig auf ein Pflanzenkulturmedium gegeben, das Mineralsalze, Vitamine, eine Kohlenhydratquelle und Hormone enthält, wobei das Hormon nach dem Geschlechtstyp der zu kultivierenden Pflanze ausgewählt wird. Die Konzentration an Auxinen kann von 0 bis 3x10⁻⁵M in Kombination mit Cytokininen variieren. Die Konzentration der Cytokinine kann ebenfalls von 0 bis 3x10⁻⁵M variieren. Gibberelline können, müssen aber nicht dem Kulturmedium zugesetzt werden. Es ist möglich, natürliche und künstliche Analoge dieser Hormone einzusetzen. Eine besondere Ausgestaltung der Erfindung besteht im Zusatz von Kokosmilch zum Kulturmedium, gegebenenfalls in Kombination mit Hormonen.

Die Behälter mit den geimpften Ovarienstückchen werden nun unter kontrollierten Bedingungen kultiviert, wobei die Temperaturen in der Regel zwischen 20 und 30°C und die Lichtintensität zwischen 500 und 5000 lux liegen. Bevorzugt ist ein 16 Stunden hell-, 8 Stunden dunkel-Zyklus und eine Beleuchtung von 1000 lux mit Leuchtstofflampen (weißes Tageslicht) und einer Temperatur von 27°C.

Nach 2 Wochen bis 3 Monaten wachsen aus den Kulturmedien Calli oder Embryonen, die anschließend auf dasselbe oder ein anderes Kulturmedium zur Regeneration in voll entwickelte Pflanzen überführt werden. Die meisten der Pflanzen, die aus diesen Embryonen hervorgehen, sind haploid, wenn auch gelegentlich diploide Embryonen beobachtet werden, die haploiden Ursprungs sind. Gewebsuntersuchungen zeigen, daß die Pflänzchen aus Zellen des Embryosacks stammen oder zumindest aus einer Megaspore nach Ablauf der Meiose.

Die Aktivität der Embryoproduktion hängt vom Geschlechtstyp der Donorpflanze und vom Kulturmedium ab. Die Zusammensetzung der Hormone erfolgt hierbei in Abstimmung auf den Geschlechtstyp: parthenocarpe Gurken erfordern in der Regel weniger Auxine als nicht-parthenocarpe zur Entwicklung haploider Embryonen. Im Mittel erhält man bei parthenocarpen Gurken einen haploiden Embryo aus zwei kultivierten Ovarien. Diese haploiden Embryonen können sich auf üblichen Kulturmedien zu Pflanzen entwickeln, beispielsweise auf Miller-Medium oder auf dem Medium nach Murashige und Skoog (1962) Physiologica Plantarum 15, 473.

Die Chromosomenverdopplung kann spontan oder nach Induktion erfolgen, wobei die üblichen Verfahren wie die Behandlung mit Colchicin Anwendung finden können. Doppelt-haploide Pflanzen werden unter Gewächshausbedingungen verpflanzt und einem Züchtungsprogramm zugeführt.

Nach dem erfindungsgemäßen Verfahren werden bis zu 80 % haploide Embryos, bezogen auf überimpfte Ovarien erhalten, abhängig vom Genotyp der Donorpflanze. Da eine Person pro Tag mindestens 300 Ovarien bearbeiten kann, können so bis zu etwa 240 Embryonen haploiden Ursprungs gewonnen werden. Die Erfindung stellt somit einen enormen Fortschritt für Pflanzenzüchtungsprogramme dar.

In dem folgenden Beispiel beziehen sich Prozentangaben auf das Gewicht, sofern keine anderen Angaben gemacht sind.

### Beispiel

Etwa 1 bis 2 Tage vor dem Aufblühen der Blüten werden aus den Fruchtknoten von Blütenknospen unter aseptischen Bedingungen Ovarien isoliert und bei 27-28°C und 2000 Lux auf dem folgenden Medium kultiviert:

### Mineralsalze:

Hauptnährsalze und Spurennährsalze wie von Miller (Miller, C.O.: Kinetin and kinetin-like compounds (1963) in: Peach, K. und Tracey, M.V., Herausgeber: Moderne Methoden in der Pflanzenanalyse, Bd. 6, 194-202, Berlin; Springer) als Medium A beschrieben.

### FeEDTA, 1x10⁻⁴M

Organische Medienzusätze nach Fujii (Clapham, D. (1971) In vitro development of callus from pollen of Lolium and Hordeum, Z. Pflanzenzüchtg., 65, 285-292)
Saccharose, 30 g
Thiamin-Hydrochlorid, 9 mg/l
6-Benzyl-aminopurin, 0,79 mg/l
Der pH-Wert des Mediums wird vor Zugabe des Agars (0,5 % Difco Bacto-Agar) auf 5,8 eingestellt. Das Medium wird durch 20 Minuten Autoklavieren bei 115°C sterilisiert. Sobald das Medium auf 55 bis 45°C abgekühlt ist, wird es in Petrischalen gegossen und nach vollständigem Abkühlen und Erstarren des Agars verwendet.

Nach 3 - 6 Wochen werden die haploiden Pflanzen sichtbar, die auf einem analogen Medium, bei dem jedoch Cytokinin weggelassen wurde, weiter kultiviert.

## Patentansprüche

1. Verfahren zur Herstellung doppelt-haploider Cucumis sativus-Pflanzen, dadurch gekennzeichnet, daß man
- unreife oder unbefruchtete Eizellen (Ovulae) oder
Zellen vom Embryosack oder
von ovarialem Gewebe umgebene unreife oder unbefruchtete Eizellen oder von ovarialem Gewebe umgebene Zellen vom Embryosack isoliert,
- die Bildung von Embryonen mit Hilfe eines hormonhaltigen Mediums induziert, das bei parthenocarpen Früchten Cytokine und bei nicht partenocarpen Früchten Auxine enthält,
- die entstandenen Embryonen zu Pflanzen regeneriert und die Duplizierung des Genoms, falls letzteres nicht spontan erfolgt, bewirkt.

2. Verfahren nach Anspruch 1, dadurch gekennzeichnet, daß man aus gegebenenfalls von Fruchtgewebe umgebenen Ovarien-Stückchen Embryos kultiviert.

3. Verfahren nach Anspruch 1 oder 2, dadurch gekennzeichnet, daß als Medium ein anorganische Salze enthaltendes Miller-Medium dient.

## Claims

1. A method for producing double-haploid Cucumis sativus plants, which comprises
- isolating immature or unfertilized ovulae, or embryo sac cells, or immature or unfertilized ovulae or embryo sac cells contained in ovarial tissue,
- inducing the formation of embryos with the aid of a hormone-containing medium, which, in the case of parthenocarpous fruit, contains cytokinins, and in the case of non-parthenocarpous fruit, the medium contains auxins,
- regenerating the embryos formed to give plants, and effecting duplication of the genome if this does not happen spontaneously.

2. The method as claimed in claim 1, wherein embryos are cultured from ovary sections which may be contained in fruit tissue.

3. The method as claimed in claim 1 or 2, wherein a Miller medium containing inorganic salts is used as the medium.

## Revendications

1. Procédé pour la préparation de plantes de *Cucumis sativus* double-haploïdes caractérisé en ce que
- on isole
- des ovules immatures ou non fécondés ou des cellules de la poche embryonnaire, ou
- des ovules immatures ou non fécondés à partir du tissu ovarien les entourant, ou
- des cellules de la poche embryonnaire à partir du tissu ovarien les entourant,
- on induit la formation des embryons à l'aide d'un milieu contenant des hormones, qui contient, dans le cas de fruits parthénocarpes des cytokines, et, dans le cas de fruits non parthénocarpes, des auxines,
- on régénère les embryons formés pour obtenir des plantes et on effectue la duplication du génome, si cette dernière ne se produit pas spontanément.

2. Procédé selon la revendication 1, caractérisé en ce qu'on cultive des embryons éventuellement à partir des morceaux d'ovaires, éventuellement entourés de tissu fécond.

3. Procédé selon les revendications 1 ou 2, caractérisé en ce que, en tant que milieu, on se sert de milieu de Miller contenant un sel minéral.
